(19) Europäisches Patentamt
European Patent Office
Office européen des brevets

(11) **EP 3 329 843 B1**

(12) **EUROPEAN PATENT SPECIFICATION**

(45) Date of publication and mention
of the grant of the patent:
**29.04.2020 Bulletin 2020/18**

(51) Int Cl.:
*A61B 5/00* *(2006.01)*       *A61B 8/08* *(2006.01)*
*A61B 5/02* *(2006.01)*

(21) Application number: **17204406.7**

(22) Date of filing: **29.11.2017**

(54) **DISPLAY CONTROL APPARATUS, DISPLAY CONTROL METHOD, AND PROGRAM**

ANZEIGESTEUERUNGSVORRICHTUNG, ANZEIGESTEUERUNGSVERFAHREN UND PROGRAMM

APPAREIL DE COMMANDE D'AFFICHAGE, PROGRAMME DE COMMANDE D'AFFICHAGE ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2016 JP 2016233208**

(43) Date of publication of application:
**06.06.2018 Bulletin 2018/23**

(73) Proprietor: **Canon Kabushiki Kaisha**
**Tokyo 146-8501 (JP)**

(72) Inventors:
• **ABE, Hiroshi**
**Ohta-ku**
**Tokyo 146-8501 (JP)**
• **OKA, Kazuhito**
**Ohta-ku**
**Tokyo 146-8501 (JP)**

(74) Representative: **Houle, Timothy James**
**Canon Europe Ltd**
**European Patent Department**
**3 The Square**
**Stockley Park**
**Uxbridge, Middlesex UB11 1ET (GB)**

(56) References cited:
**EP-A1- 2 853 917       US-A1- 2014 196 544**
**US-A1- 2014 360 271       US-A1- 2015 043 800**
**US-A1- 2015 073 278**

**Description**

BACKGROUND OF THE INVENTION

Field of the Invention

The present invention relates to an apparatus that controls display of photoacoustic image data.

Description of the Related Art

Photoacoustic imaging is an imaging technology in which a photoacoustic wave generated from an optical absorber irradiated with light is received, and a spatial distribution of the optical absorber can be imaged. When the photoacoustic imaging is applied to a living body, the optical absorber such as a blood vessel including hemoglobin can be imaged.

According to Japanese Patent Laid-Open No. 2013-233386, photoacoustic image data in a three-dimensional (3D) space (XYZ space) is generated by using a photoacoustic imaging principle, and a tomographic image of the photoacoustic image data on a certain plane is displayed. According to Japanese Patent Laid-Open No. 2013-233386, a plurality of ultrasonic transducers including probes arranged in an X-direction are provided, and a tomographic image of the photoacoustic image data in an XZ cross section is displayed in a case where scanning of the probes is performed in a Y-direction.

US 2014/196544 A1 discusses an object information acquiring apparatus includes a detector configured to detect a photoacoustic wave generated from an object on which light is irradiated from a light source, a generator configured to generate, using the photoacoustic wave, an image indicating characteristic information in a photographing region in the object, an acquirer configured to acquire information concerning quantitativity of a measurement value of the photoacoustic wave in the photographing region, and an extractor configured to extract a region having quantitativity in the image on the basis of the information concerning quantitativity.

SUMMARY OF THE INVENTION

However, in a case where a certain plane in a three-dimensional space is set as a display cross section when a tomographic image of photoacoustic image data is displayed, an image having a low visibility may be obtained in some cases.

In view of the above, the present invention provides an apparatus that can display an image of the photoacoustic image data having a high visibility.

The present invention in its first aspect provides a display control apparatus as specified in claims 1 to 13.

The present invention in its second aspect provides a display control method as specified in claim 14.

The present invention in its third aspect provides a program as specified in claim 15.

Further features of the present invention will become apparent from the following description of exemplary embodiments with reference to the attached drawings.

BRIEF DESCRIPTION OF THE DRAWINGS

Figs. 1A and 1B are schematic diagrams illustrating a display cross section according to an exemplary embodiment of the present invention and a comparative example.

Fig. 2 is a block diagram illustrating a photoacoustic apparatus according to the present exemplary embodiment.

Figs. 3A and 3B are schematic diagrams illustrating a probe according to the present exemplary embodiment.

Fig. 4 is a block diagram illustrating a configuration of a computer and its surrounding according to the present exemplary embodiment.

Fig. 5 is a flow chart illustrating a display method according to the present exemplary embodiment.

Figs. 6A and 6B are schematic diagrams illustrating a generation method for an image according to the present exemplary embodiment.

Figs. 7A, 7B, and 7C are schematic diagrams illustrating another generation method for the image according to the present exemplary embodiment.

Figs. 8A and 8B are schematic diagrams illustrating the generation method for the image according to the present exemplary embodiment.

Figs. 9A and 9B are schematic diagrams illustrating a graphical user interface (GUI) according to the present exemplary embodiment.

DESCRIPTION OF THE EMBODIMENTS

An aspect of the present invention is related to a method of displaying photoacoustic image data as volume data derived from a photoacoustic wave generated by light irradiation of an object. The photoacoustic image data is the volume data representing a three-dimensional spatial distribution of at least one piece of object information such as a generated sound pressure (initial sound pressure), an optical absorption energy density, and an optical absorption coefficient of the photoacoustic wave, and a concentration of a material constituting the object (such as an oxygen saturation).

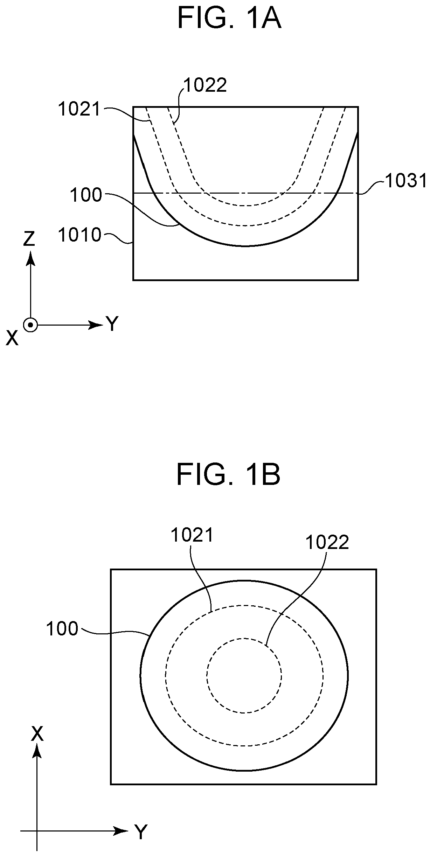

Fig. 1A is a schematic diagram of the photoacoustic image data as reconstructed volume data in which a rectangular reconstruction region 1010 is set. The photoacoustic image data illustrated in Fig. 1A includes image data of an object 100. The photoacoustic image data illustrated in Fig. 1A is obtained by irradiation of light from a lower direction along the plane of the paper (e.g. along the Z-axis direction).

A light fluence of irradiation light inside the object 100 typically attenuates at an exponential manner from a surface of the object 100. For this reason, typically, the sound pressure of the generated photoacoustic wave tends to decrease as a distance from the surface of the object 100 increases, and a signal-to-noise (S/N) ratio of the photoacoustic image data tends to decrease as the distance from the surface of the object 100 increases. Quasi isosurfaces (where values are substantially equal to one another) of the light fluence are represented by dotted lines 1021 and 1022 in Fig. 1A. The quasi isosurface represented by the dotted line 1021 represents a surface having a higher light fluence than that of the quasi isosurface represented by the dotted line 1022. It should be noted that, as described above, the quasi isosurfaces of the light fluences tend to have the shape as the surface-shape of the object 100 or a similar shape as the surface-shape of the object 100.

A case of an application of a multi planar reconstruction (MPR) for displaying a tomographic image by cutting out the photoacoustic image data on an arbitrary plane, with respect to the photoacoustic image data having the above-described characteristics, will be considered as a comparative example. A tomographic image illustrated in Fig. 1B is displayed in a case where the photoacoustic image data is cut out on a plane 1031 represented by a dashed-dotted line in Fig. 1A. As illustrated in Fig. 1B, a large variation occurs in light fluences reaching respective positions on the plane 1031. For this reason, since a large variation occurs in the S/N ratios in the tomographic image and a large variation also occurs in image qualities, the visibility of the tomographic image is low. That is, the tomographic image including a redundant region having a low image quality is displayed.

In view of the above, the inventor of the present invention has discovered that photoacoustic image data on a surface having the shape as the surface-shape of the object 100 or the similar shape as the surface-shape of the object 100, can be extracted to generate and display an image of the photoacoustic image data that addresses the above concerns. As a result, it is possible to generate an image of the photoacoustic image data in which a quasi isosurface of the light fluences, corresponding to a surface having the shape as the surface-shape of the object 100 or the similar shape as the surface-shape of the object 100, is set as the display cross section. For this reason, an image having a small variation in S/N ratio at the respective positions in the image and a high visibility is displayed. As a result, a user such as a doctor can conduct a diagnosis by checking the image having the small variation in the image qualities in the image, and a diagnostic performance is improved. In a case where the object 100 is a living body such as a breast, the surface having the shape as the surface-shape of the object 100 or the similar shape as the surface-shape of the object 100 typically is a curved surface.

Images on a plurality of surfaces having the shape as the surface-shape of the object 100 or the similar shape as the surface-shape of the object 100 may be generated and displayed. An image of the photoacoustic image data in a normal direction of the surface of the object 100 may be transmitted and displayed. That is, a plurality of images having the shape as the surface-shape of the object 100 or the similar shape as the surface-shape of the object 100 may be sequentially displayed at time points determined on the basis of a predetermined time interval or a user instruction. For example, an image obtained by cutting out the photoacoustic image data of the quasi isosurface 1021 and an image obtained by cutting out the photoacoustic image data of the quasi isosurface 1022 may be sequentially displayed.

In addition, the image having the shape as the surface-shape of the object 100 or the similar shape as the surface-shape of the object 100 may be displayed in a case where volume data (three-dimensional image data) obtained by another modality such as an ultrasonic diagnosis apparatus, computed tomography (CT), or magnetic resonance imaging (MRI) is displayed together with the photoacoustic image data. In this case, display may be performed such that the image of the photoacoustic image data can be compared with the image of the image data obtained by the other modality by displaying the images in parallel, superimposing, switching, or the like. It should be noted that, in a case where shapes of the object 100 in the photoacoustic image data and the image data obtained by the other modality are varied, at least one of the image data may be altered to reduce a difference in shapes. After alteration processing is performed, the images of the respective image data on the surface having the shape as the surface-shape of the object

100 or the similar shape as the surface-shape of the object 100 may be generated and displayed such that the images can be compared with each other.

It should be noted that an image having the shape as the surface-shape of the object 100 or the similar shape as the surface-shape of the object may be generated and displayed by projecting photoacoustic image data corresponding to a layer having a thickness, where the thickness has the shape as the surface-shape of the object 100 or the similar shape as the surface-shape of the object 100. At this time, a two-dimensional projection image on the surface having the shape as the surface-shape of the object 100 or the similar shape as the surface-shape of the object may be generated by performing the projection in the normal direction of the surface of the object. According to the above-described projection method, since spatial distributions of the light fluences in respective projection directions become comparative with one another, the variation in the S/N ratios in the image is reduced. The two-dimensional projection image obtained by projecting the photoacoustic image data corresponding to the layer having the thickness, where the thickness has the shape as the surface-shape of the object 100 or the similar shape as the surface-shape of the object 100, as described above may be set as the image of the photoacoustic image data on the surface having the surface-shape of the object. It should be noted that the layer having the thickness, where the thickness has the shape as the surface-shape of the object 100 or the similar shape as the surface-shape of the object 100, may be a layer where the surface having the shape as the surface-shape of the object 100 or the similar shape as the surface-shape of the object passes through a center or may also be a layer where the surface having the shape as the surface-shape of the object 100 or the similar shape as the surface-shape of the object constitutes any surface.

It should be noted that the example has been described with reference to Figs. 1A and 1B where the light irradiation is performed towards a wide region from the lower direction along the plane of the paper (e.g. along the Z-axis direction), but it is also possible to perform the image display on the basis of the above-described display method in a case where the light irradiation is performed by using another method. For example, the image display method according to the exemplary embodiment of the present invention may be employed in a case where the light irradiation is locally performed or a case where the light irradiation is performed at a plurality of positions.

It should be noted that the image of the photoacoustic image data may be an image obtained by allocating a luminance to an image value of the photoacoustic image data, or may also be an image obtained by allocating the luminance to a value obtained by multiplying the image value of the photoacoustic image data by a coefficient. In addition, the image of the photoacoustic image data may be an image obtaining by allocating a hue, a brightness, or a saturation to the image value of the photoacoustic image data or a value obtained by multiplying the value by a coefficient. That is, the image of the photoacoustic image data may be an image represented in any manner as long as the image is based on the image value of the photoacoustic image data.

Hereinafter, an exemplary embodiment of the present invention will be described with reference to the drawings. It should be noted however that dimensions, materials, and shapes of components which will be described below, those relative positions, and the like are to be appropriately changed depending on the configurations and various conditions of the apparatus to which the exemplary embodiment of the present invention is applied, and are not intended to limit the scope of the present invention to the following descriptions. Each of the embodiments of the present invention described below can be implemented solely or as a combination of a plurality of the embodiments or features thereof where necessary or where the combination of elements or features from individual embodiments in a single embodiment is beneficial.

Hereinafter, a configuration of a photoacoustic apparatus according to the present exemplary embodiment and an information processing method will be described.

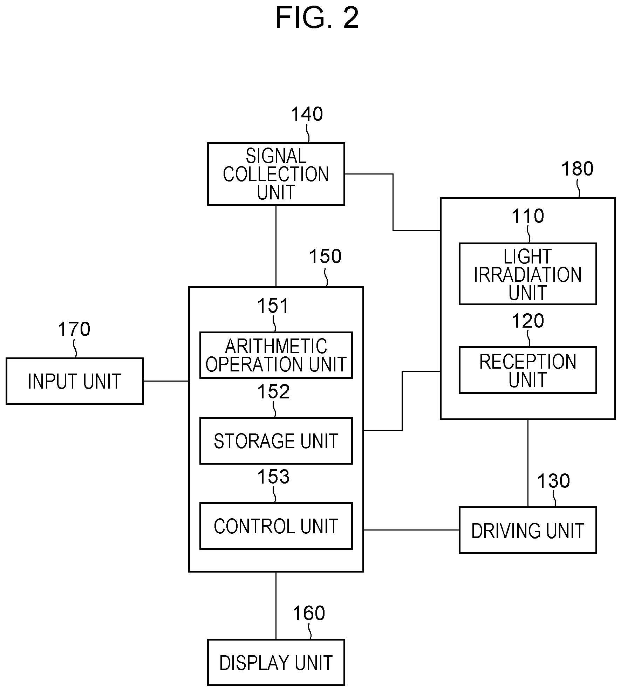

According to the present exemplary embodiment, an example using a photoacoustic apparatus will be described. The configuration of the photoacoustic apparatus according to the present exemplary embodiment will be described with reference to Fig. 2. Fig. 2 is a schematic block diagram of the entirety of the photoacoustic apparatus. The photoacoustic apparatus according to the present exemplary embodiment includes a probe 180 including a light irradiation unit 110 and a reception unit 120, a driving unit 130, a signal collection unit 140, a computer 150, a display unit 160, and an input unit 170.

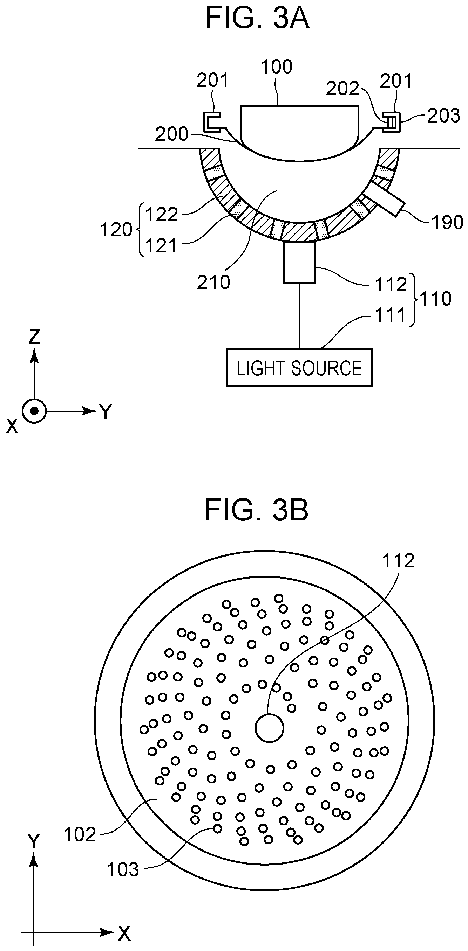

Figs. 3A and 3B are schematic diagrams of the probe 180 according to the present exemplary embodiment. A measurement object is the object 100. The driving unit 130 drives the light irradiation unit 110 and the reception unit 120 and performs mechanical scanning. The light irradiation unit 110 irradiates the object 100 with light, and an acoustic wave is generated in the object 100. The acoustic wave generated by a photoacoustic effect derived from the light is also referred to as a photoacoustic wave. The reception unit 120 outputs an electric signal (photoacoustic signal) as an analog signal when the photoacoustic wave is received.

The signal collection unit 140 converts the analog signal output from the reception unit 120 into a digital signal to be output to the computer 150. The computer 150 stores the digital signal output from the signal collection unit 140 as signal data derived from an ultrasonic wave or the photoacoustic wave.

The computer 150 generates the volume data (photoacoustic image) representing information (object information) related to the object 100 by performing signal processing on the stored digital signal. In addition, the computer 150 causes the display unit 160 to display an image of the obtained volume data. The doctor acting as the user can perform the diagnosis by checking the image displayed on the display unit 160. The display image is saved in a memory in the computer 150, a data management system connected to a modality by a network, or the like on the basis of a saving instruction from the user or the computer 150.

The computer 150 also performs driving control on the components included in the photoacoustic apparatus. The display unit 160 may also display a graphical user interface (GUI) or the like in addition to the image generated by the computer 150. The input unit 170 is configured such that the user can input information. The user can perform operations such as measurement start and end and the saving instruction of the generated image by using the input unit 170.

Hereinafter, details of the respective components of the photoacoustic apparatus according to the present exemplary embodiment will be described.

Light irradiation unit 110

The light irradiation unit 110 includes a light source 111 that emits light and an optical system 112 that guides the light emitted from the light source 111 to the object 100. It should be noted that the light includes pulse light such as a so-called rectangular wave or chopping wave.

A pulse width of the light emitted from the light source 111 may be a pulse width larger than or equal to 1 ns and smaller than or equal to 100 ns. A wavelength in a range between approximately 400 nm to approximately 1600 nm may be set as a wavelength of the light. A wavelength (which is higher than or equal to 400 nm and lower than or equal to 700 nm) at which absorption in the blood vessel is high may be used in a case where imaging of the blood vessel is performed at a high resolution. Light at a wavelength (which is higher than or equal to 700 nm and lower than or equal to 1100 nm) at which absorption in a background tissue (such as water or fat) of the living body is typically low may be used in a case where imaging of a deep part of the living body is performed.

A laser or a light emitting diode can be used as the light source 111. When measurement is performed by using light at a plurality of wavelengths, a light source that can change the wavelength may also be used. It should be noted that, in a case where the object is irradiated with the plurality of wavelengths, a plurality of light sources that generate light having mutually different wavelengths can be prepared, and the light is alternately emitted from the respective light sources. Even in a case where the plurality of light sources are used, those light sources are collectively represented as the light source. Various lasers including a solid-state laser, a gas laser, a dye laser, and a semiconductor laser can be used as the laser. For example, a pulse laser such as an Nd:YAG laser and an alexandrite laser may be used as the light source. In addition, a Ti:sa laser or an optical parametric oscillator (OPO) using Nd:YAG laser light as exciting light laser may be used as the light source. Moreover, a flash lamp or a light emitting diode may be used as the light source 111. Furthermore, a microwave source may be used as the light source 111.

An optical element such as a lens, a mirror, or an optical fiber can be used as the optical system 112. In a case where the breast or the like is set as the object 100, to perform the irradiation by widening a beam diameter of the pulse light, a light outgoing part of the optical system 112 may be constituted by a diffusing plate or the like that diffuses the light. On the other hand, to increase the resolution, the light outgoing part of the optical system 112 may be constituted by a lens or the like, and the irradiation may be performed while the beam is focused in a photoacoustic microscope.

It should be noted that the light irradiation unit 110 may directly irradiate the object 100 with light from the light source 111 without the provision of the optical system 112.

Reception unit 120

The reception unit 120 includes transducers 121 that output an electric signal when the acoustic wave is received and a supporting member 122 that supports the transducers 121. A transmission unit that transmits an acoustic wave may be set as the transducer 121. A transducer serving as a reception unit and the transducer serving as the transmission unit may be a single (common) transducer or may also be separate components.

A piezo-ceramic material represented by lead zirconate titanate (PZT), a polymer piezoelectric membrane material represented by polyvinylidene-fluoride (PVDF), or the like can be used as a member constituting the transducer 121. An element other than a piezoelectric element may also be used. For example, a capacitive micromachined ultrasonic transducer (CMUT), a transducer using a Fabry-Perot interferometer, or the like can be used. It should be noted that any transducer may be adopted as long as the transducer can output the electric signal when the acoustic wave is received. The signal obtained by the transducer is a time-resolved signal. That is, an amplitude of the signal obtained by the transducer represents a value based on a sound pressure received by the transducer at each time (for example, a value in proportion to the sound pressure).

A frequency component constituting the photoacoustic wave is typically 100 KHz to 100 MHz, and it is possible to adopt an element that can detect these frequencies as the transducer 121.

The supporting member 122 may be formed of a metallic material having a high mechanical strength or the like. A surface on a side of the object 100 of the supporting member 122 may be processed to have a mirror surface or realize light scattering such that much irradiation light enters the object. According to the present exemplary embodiment, the supporting member 122 has a shape of a hemispherical enclosure and is constituted such that the plurality of transducers 121 can be supported on the hemispherical enclosure. In this case, directional axes of the transducers 121 arranged in the supporting member 122 converge in the vicinity of the center of curvature of the hemispherical enclosure. An image quality in the vicinity of the center of curvature is increased when the imaging is performed by using the signals output from the plurality of transducers 121. It should be noted that the supporting member 122 may adopt any configuration as long as the supporting member 122 can support the transducers 121. The plurality of transducers may be disposed and arranged in a plane or a curved-surface such as a so-called 1D array, 1.5D array, 1.75D array, or 2D array in the supporting member 122. The plurality of transducers 121 are equivalent to a plurality of reception units.

The supporting member 122 may also function as a container that retains an acoustic matching material 210. That is, the supporting member 122 may be constituted by a container that arranges the acoustic matching material 210 between the transducer 121 and the object 100.

The reception unit 120 may include an amplifier that amplifies a time-series analog signal output from the transducer 121. The reception unit 120 may also include an analog-to-digital converter that converts the time-series analog signal output from the transducer 121 into a time-series digital signal. That is, the reception unit 120 may include the signal collection unit 140 which will be described below.

It should be noted that the transducers 121 may be ideally arranged so as to surround the object 100 from the entire circumference such that the acoustic waves can be detected at various angles. It should be noted however that, in a case where the transducers are not arranged so as to surround the object 100 from the entire circumference because the object 100 is large, the transducers may be arranged on the hemispherical supporting member 122 to substantially establish a state in which the object 100 is surrounded from the entire circumference.

It should be noted that the arrangement and the number of the transducers and the shape of the supporting member may be optimized in accordance with the object, and any type of the reception unit 120 can be adopted with regard to the exemplary embodiment of the present invention.

A space between the reception unit 120 and the object 100 is filled with a medium with which the photoacoustic wave propagates. By using a material in which the acoustic wave can propagate, acoustic characteristics are matched on an interface between the object 100 and the transducer 121, and a material that allows transmittance of the photoacoustic wave as high as possible is adopted. For example, water, ultrasonic gel, or the like may be adopted as the material.

Fig. 3A is a lateral view of the probe 180, and Fig. 3B is a top view of the probe 180 (viewed from an upward direction along the plane of the paper in Fig. 3A (e.g. along the Z-axis direction)). The probe 180 according to the present exemplary embodiment illustrated in Fig. 2 includes the reception unit 120 in which the plurality of transducers 121 are three-dimensionally arranged in the hemispherical supporting member 122 having openings. The light outgoing part of the optical system 112 is arranged in a bottom part of the supporting member 122 in the probe 180 illustrated in Fig. 2.

According to the present exemplary embodiment, as illustrated in Fig. 2, while the object 100 is in contact with a holding part 200, a shape of the object 100 is maintained. According to the present exemplary embodiment, in a case where the object 100 is a breast, a mode is presumed in which a bunk (or table) that supports an examinee in a prone position is provided with an opening for inserting the breast, and the breast suspended in a vertical direction through the opening is measured.

A space between the reception unit 120 and the holding part 200 is filled with a medium (the acoustic matching material 210) in which the photoacoustic wave can propagate. By using a material in which the acoustic wave can propagate, the acoustic characteristics are matched on the interface between the object 100 and the transducer 121, and a material that allows the transmittance of the photoacoustic wave as high as possible is adopted. For example, water, ultrasonic gel, or the like may be adopted as this medium.

The holding part 200 as a holding unit is used for holding the shape of the object 100 during the measurement. While the holding part 200 holds the object 100, a movement of the object 100 can be suppressed, and the position of the object 100 can be kept in the holding part 200. A resin material such as polycarbonate, polyethylene, or polyethylene terephthalate can be used as a material of the holding part 200.

The holding part 200 is preferably formed of a material having a firmness to such an extent that the object 100 can be held. The holding part 200 may be formed of a material through which the light used in the measurement transmits. The holding part 200 may be formed of a material in which an impedance is at a comparable level with that of the object 100. In a case where on object having a curvature of the breast or the like is set as the object 100, the holding part 200 molded to have a concave shape may also be adopted. In this case, the object 100 can be inserted into a concave part of the holding part 200.

The holding part 200 is attached to a fitting part 201. The fitting part 201 may be constituted in a manner that a plurality of types of the holding parts 200 can be replaced in accordance with the size of the object. For example, the fitting part 201 may also be constituted in a manner that holding parts having different radii of curvature, centers of curvature, or the like can be replaced.

A tag 202 in which information of the holding part 200 is registered may be installed in the holding part 200. For example, it is possible to register information such as the radius of curvature or the center of curvature of the holding part 200, acoustic velocity, or a discrimination ID in the tag 202. The information registered in the tag 202 is read out by a reading unit 203 to be transferred to the computer 150. To easily read the tag 202 when the holding part 200 is attached to the fitting part 201, the reading unit 203 may be installed in the fitting part 201. For example, the tag 202 is a barcode, and the reading unit 203 is a barcode reader. Driving unit 130

The driving unit 130 is a part that changes a relative position of the object 100 and the reception unit 120. According to the present exemplary embodiment, the driving unit 130 is an apparatus that moves the supporting member 122 in an XY direction and is an electrically-driven XY stage to which a stepping motor is mounted. The driving unit 130 includes a motor such as the stepping motor that generates driving force, a driving mechanism that transmits the driving force, and a positional sensor that detects positional information of the reception unit 120. A lead screw mechanism, a link mechanism, a gear mechanism, an oil pressure mechanism, or the like can be used as the driving mechanism. A potentiometer or the like using an encoder, a variable resistor, or the like can be used as the positional sensor.

It should be noted that the driving unit 130 may not only change the relative position of the object 100 and the reception unit 120 in the XY direction (two dimensions) but also change one-dimensionally or three-dimensionally. A movement path may be two-dimensionally scanned in a spiral shape or a line and space manner, and furthermore, the movement path may be three-dimensionally inclined along a body surface. In addition, the probe 180 may be moved so as to keep a constant distance from the surface of the object 100. At this time, the driving unit 130 may measure the movement amount of the probe by monitoring the number of revolutions of the motor or the like.

It should be noted that the driving unit 130 may fix the reception unit 120 and move the object 100 as long as the relative position of the object 100 and the reception unit 120 can be changed. A configuration in which the object 100 is moved by moving the holding part that holds the object 100 or the like is conceivable in a case where the object 100 is moved. Both the object 100 and the reception unit 120 may also be moved.

The driving unit 130 may continuously move the relative position or may move the relative position by a step and repeat manner. The driving unit 130 may be an electrically-driven stage that moves the relative position on a programmed track or a manually-operated stage. That is, the photoacoustic apparatus may be of a hand-held type in which the user performs the operation by holding the probe 180 without the provision of the driving unit 130.

In addition, according to the present exemplary embodiment, the driving unit 130 simultaneously drives the light irradiation unit 110 and the reception unit 120 to perform the scanning, but only the light irradiation unit 110 may be driven, and also only the reception unit 120 may be driven.

Signal collection unit 140

The signal collection unit 140 includes an amplifier that amplifies the electric signal corresponding to the analog signal output from the transducer 121, and an A/D converter that converts the analog signal output from the amplifier into a digital signal. The signal collection unit 140 may be constituted by a field programmable gate array (FPGA) chip or the like. The digital signal output from the signal collection unit 140 is stored in the storage unit 152 in the computer 150. The signal collection unit 140 is also referred to as a data acquisition system (DAS). The electric signal in the present specification is a concept including both of the analog signal and the digital signal. It should be noted that the signal collection unit 140 may be connected to a light detection sensor attached to the light outgoing part of the light irradiation unit 110, and start processing in synchronism with the light emitted from the light irradiation unit 110 as a trigger. In addition, the signal collection unit 140 may start the processing in synchronism with an instruction issued by using a freeze button or the like as a trigger.

Computer 150

The computer 150 serving as a display control apparatus includes an arithmetic operation unit 151, a storage unit 152, and a control unit 153. Functions of the respective configurations will be described when a processing flow will be described.

Units realizing an arithmetic operation function as the arithmetic operation unit 151 can be constituted by a processor such as a CPU or a graphics processing unit (GPU) or an arithmetic operation circuit such as a field programmable gate array (FPGA) chip. These units may be constituted by not only a single processor or arithmetic operation circuit but also a plurality of processors or arithmetic operation circuits. The arithmetic operation unit 151 may receive various parameters such as the object acoustic velocity or the configuration of the holding part from the input unit 170 and process the reception signal.

The storage unit 152 can be constituted by a read only memory (ROM) or a non-transitory storage medium such as a magnetic disc or a flash memory. The storage unit 152 may also be a volatile medium such as a random access memory (RAM). It should be noted that the storage medium that stores the program is the non-transitory storage medium. It should also be noted that the storage unit 152 may be not only constituted by a single storage medium but also constituted by a plurality of storage media.

The storage unit 152 can save image data indicating the photoacoustic image generated by the arithmetic operation unit 151 by a method which will be described below.

The control unit 153 is constituted by an arithmetic operation element such as a CPU. The control unit 153 controls operations of the respective components of the photoacoustic apparatus. The control unit 153 may receive instruction signals based on various operations such as measurement start from the input unit 170, and control the respective components of the photoacoustic apparatus. The control unit 153 also reads out program codes stored in the storage unit 152 and controls actions of the respective components of the photoacoustic apparatus.

The computer 150 may be a dedicatedly designed work station. Respective configurations of the computer 150 may be constituted by different hardware components. In addition, at least part of the configurations of the computer 150 may be constituted by a single piece of hardware.

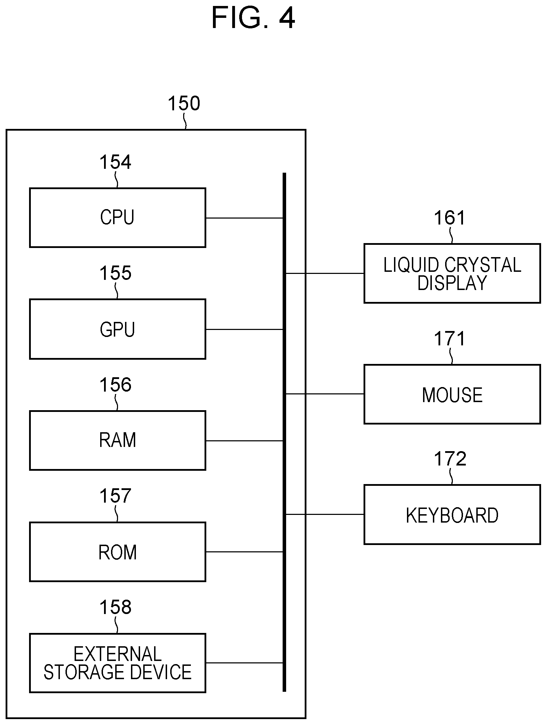

Fig. 4 illustrates a specific configuration example of the computer 150 according to the present exemplary embodiment. The computer 150 according to the present exemplary embodiment is constituted by a CPU 154, a GPU 155, a RAM 156, a ROM 157, and an external storage device 158. A liquid crystal display 161 functioning as the display unit 160 and a mouse 171 and a keyboard 172 functioning as the input unit 170 are connected to the computer 150.

The computer 150 and the plurality of transducers 121 may be provided by a configuration of being contained in a common casing. It should be noted however that the computer contained in the casing may perform part of the signal processing, and a computer installed outside the casing may perform the rest of the signal processing. In this case, the computers installed inside and outside the casing can be collectively referred to as the computer according to the present exemplary embodiment. That is, it is sufficient even when hardware components constituting the computer are not contained in the single casing. Display unit 160

The display unit 160 is a display such as a liquid crystal display, an organic electro luminescence (EL) FED, a spectacle display, or a head mounted display. The display unit 160 is an apparatus that displays an image based on the object information or the like obtained by the computer 150, a numeric value of a specific position, or the like. The display unit 160 may display a GUI for operating the image or the apparatus. It should be noted that, when the object information is displayed, image processing (such as adjustment of the luminance value) may be performed in the display unit 160 or the computer 150 before the display is performed. The display unit 160 may be provided separately in addition to the photoacoustic apparatus. The computer 150 can transmit the photoacoustic image data to the display unit 160 in a wired or wireless manner.

Input unit 170

An operation console can be adopted as the input unit 170. The operation console is constituted by a mouse, a keyboard, or the like that can be operated by the user. The display unit 160 may be constituted by a touch panel, and the display unit 160 can be used as the input unit 170.

The input unit 170 may be constituted such that information of a position or a depth to be desired to be observed or the like can be input. As an input method, a numeric value may be input, or an input operation can be performed by operating a slider bar. The image to be displayed on the display unit 160 may be updated in accordance with the input information. As a result, the user can set appropriate parameters by checking at the image generated by the parameters determined by its own operation.

It should be noted that the respective components of the photoacoustic apparatus may be constituted as individual apparatuses or may be constituted as an integrated single apparatus. A configuration as a single apparatus may also be adopted in which at least part of the components of the photoacoustic apparatus is integrated.

The information transmitted and received between the respective components of the photoacoustic apparatus is exchanged in a wired or wireless manner.

Object 100

The object 100 will be described below although the object 100 does not constitute the photoacoustic apparatus. The photoacoustic apparatus according to the present exemplary embodiment can be used for a purpose of a diagnosis on malignant tumor, blood vessel disease, or the like of a human being or an animal, follow-up of a chemical treatment, or the like. Therefore, a living body, specifically, a target region of the diagnosis such as a human or animal breast, respective organs, a network of vessels, a head region, a neck region, an abdominal region, or four limbs including fingers and toes is presumed as the object 100. For example, when a human body is the measurement object, a newborn blood vessel formed in the vicinity of a blood vessel or tumor containing a large amount of oxyhemoglobin or deoxyhemoglobin or the like may be set as the target of the optical absorber. Plaque of a carotid wall or the like may also be set as the target of the optical absorber. In addition, pigment such as methylene blue (MB) or indocyanine green (ICG), fine gold particles, or a material where those materials are accumulated or a chemically modified material introduced from the outside may be set as the optical absorber.

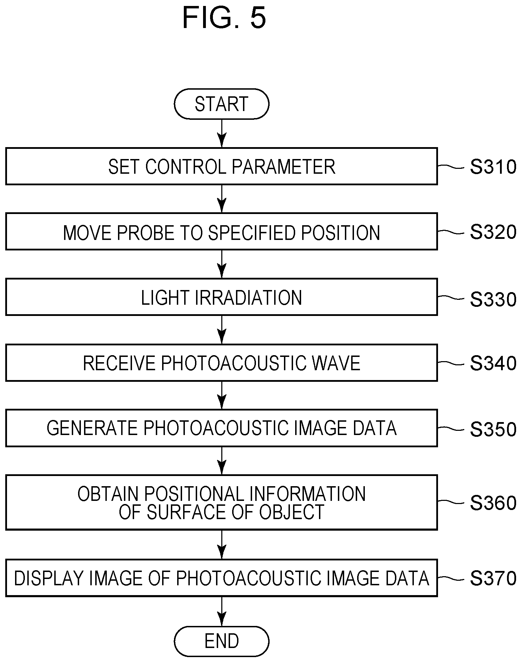

Next, a display method including information processing according to the present exemplary embodiment will be described with reference to Fig. 5. It should be noted that respective steps are executed while the computer 150 controls the operations of the components of the photoacoustic apparatus.

S310: Step of setting control parameter

The user uses the input unit 170 to specify a control parameter such as an irradiation condition (repetition frequency or wavelength) of the light irradiation unit 110 which is used for obtaining the object information or a position of the probe 180. The computer 150 sets the control parameter determined on the basis of the instruction of the user.

S320: Step of moving probe to specified position

The control unit 153 causes the driving unit 130 to move the probe 180 to a specified position on the basis of the control parameter specified in step S310. In a case where the imaging is specified in a plurality of positions in step S310, first, the driving unit 130 moves the probe 180 to an initial specified position. It should be noted that the driving unit 130 may move the probe 180 to a previously programmed position when a start instruction for measurement is issued. It should also be noted that the user may hold the probe 180 to be moved to a desired position in a case where the photoacoustic apparatus is of the hand-held type.

S330: Step of performing light irradiation

The light irradiation unit 110 irradiates the object 100 with light on the basis of the control parameter specified in Step S310.

The object 100 is irradiated with the light generated from the light source 111 via the optical system 112 as the pulse light. Subsequently, the pulse light is absorbed inside the object 100, and the photoacoustic wave is generated by the photoacoustic effect. The light irradiation unit 110 transmits a synchronization signal to the signal collection unit 140 along with the transmission of the pulse light.

S340: Step of receiving photoacoustic wave

The signal collection unit 140 starts signal collection when the synchronization signal transmitted from the light irradiation unit 110 is received. That is, the signal collection unit 140 performs amplification and AD conversion of the analog electric signal derived from the acoustic wave which is output from the reception unit 120 to generate the amplified digital electric signal to be output to the computer 150. The computer 150 saves the signal transmitted from the signal collection unit 140 in the storage unit 152. In a case where the imaging is specified in a plurality of scanning positions in step S301, the steps S320 to S340 are repeatedly executed in the specified scanning positions, and the pulse light irradiation and the generation of the digital signal derived from the acoustic wave are repeated.

S350: Step of generating photoacoustic image data

The arithmetic operation unit 151 in the computer 150 generates the photoacoustic image data as the volume data based on signal data stored in the storage unit 152 and saves the photoacoustic image data in the storage unit 152. Any techniques such as a time domain reverse projection method, a Fourier domain reverse projection method, or a model base method (repeated operation method) may be adopted as a reconstruction algorithm for converting the signal data into the three-dimensional volume data. For example, the time domain reverse projection method includes universal back-projection (UBP), filtered back-projection (FBP), phasing addition (delay-and-sum), or the like. For example, the arithmetic operation unit 151 may adopt a UBP method represented by Expression (1) as the reconstruction technology for obtaining a three-dimensional spatial distribution of a generated sound pressure (initial sound pressure) of the acoustic wave as the photoacoustic image data.

$$p_0(\mathbf{r}_0) = \frac{\sum_i^N b\left(\mathbf{r}_i, t = \frac{|\mathbf{r}_i - \mathbf{r}_0|}{c}\right) \cdot \Delta\Omega_i}{\sum_i^N \Delta\Omega_i}$$

$$b(\mathbf{r}, t) = 2p(\mathbf{r}, t) - 2t\frac{\partial p(\mathbf{r}, t)}{\partial t} \qquad (1)$$

Where $r_0$ denotes a positional vector indicating a position for performing reconstruction (also referred to as a reconstruction position or a position of interest), po ($r_0$, t) denotes an initial sound pressure in the position for performing the reconstruction, and c denotes the acoustic velocity of a propagation path. $\Delta\Omega_i$ denotes a solid angle viewing the i-th transducer 121 from the position for performing the reconstruction, and N denotes the number of transducers 121 used for the reconstruction. Expression (1) represents performance of phasing addition (reverse projection) by carrying out processing such as differentiation on reception signals p (ri, t) and applying weighting of the solid angle to those. Herein, t in Expression (1) denotes a time (propagation time) for the photoacoustic wave to propagate through an acoustic ray between the position of interest and the transducer 121. It should be noted that arithmetic operation processing may also be performed in a calculation of b (ri, t). For example, the arithmetic operation processing includes frequency filtering (low-pass, high-pass, band-pass, or the like), deconvolution, envelope demodulation, wavelet filtering, or the like.

The arithmetic operation unit 151 may also obtain absorption coefficient distribution information by calculating the light fluence distribution inside the object 100 of the light with which the object 100 is irradiated and dividing an initial sound pressure distribution by the light fluence distribution. In this case, the absorption coefficient distribution information may be obtained as the photoacoustic image data. In addition, steps S330 and S340 may be executed by using light at a plurality of wavelengths, and the arithmetic operation unit 151 may obtain the absorption coefficient distribution information corresponding to each of the light at the plurality of wavelengths. The arithmetic operation unit 151 may obtain spatial distribution information of a concentration of a material constituting the object 100 as spectroscopic information as the photoacoustic image data on the basis of the absorption coefficient distribution information corresponding to each of the light at the plurality of wavelengths.

S360: Step of obtaining positional information of object surface

The computer 150 serving as a first unit (first means) obtains positional information of the surface of the object 100 to be saved in the storage unit 152. The positional information may be coordinates of the surface themselves or may also be information representing the position of the surface by a function.

For example, the computer 150 may receive positional information of the surface of the object 100 obtained by an optical three-dimensional camera and obtain the positional information of the surface of the object 100. The computer 150 may also obtain the positional information of the surface of the object 100 on the basis of the three-dimensional image data of the object 100 imaged by the optical three-dimensional camera or another modality (such as the ultrasonic diagnosis apparatus, the CT, or the MRI). In this case, the computer 150 may obtain the positional information of the surface of the object 100 by performing image processing such as edge detection processing on the three-dimensional image data of the object 100. It should be noted that the optical three-dimensional camera or the other modality may be an apparatus integrated with the photoacoustic apparatus according to the present exemplary embodiment or may be a separate apparatus.

The computer 150 may obtain the positional information of the surface of the object 100 on the basis of the photoacoustic image data obtained in S350. In this case too, the computer 150 may obtain the positional information of the surface of the object 100 by performing the image processing such as the edge detection processing on the photoacoustic image data. For example, after a smoothing filter such as Gaussian is applied to the photoacoustic image data, the computer 150 can define the quasi isosurface in the photoacoustic image data after the smoothing as the surface of the object 100. That is, a surface continuously having substantially equal values in the volume data can be defined as the surface of the object 100. It should be noted that not only a surface constituted by completely equal values in the photoacoustic image data after the smoothing but also a surface in which a variation in the image values is within a predetermined range may be defined as the quasi isosurface. A numeric value range in which the values of the photoacoustic image data can be regarded as substantially equal to one another may be specified by the user using the input unit 170. With regard to a curved surface or a curved line corresponding to a candidate of the surface of the object 100, an order, a coefficient, or the like may be previously set in accordance with the shape of the object 100 to be imaged. As a result, it is possible to reduce processing time used for defining the surface of the object 100.

In addition, the computer 150 may perform rendering on the photoacoustic image data obtained in S350 to be displayed on the display unit 160 and obtain the positional information of the surface of the object 100 by using the displayed rendering image. For example, when the user operates the input unit 170, a plurality of positions assumed to be the surface of the object 100 are clicked in the rendering image. Subsequently, the computer 150 may interpolate and connect those positions to define the surface and obtain the positional information of the surface of the object 100. In addition, when the user directly inputs coordinates of the positions assumed to be the surface of the object 100 while the rendering image is checked, the computer 150 may obtain the positional information of the surface of the object 100. A desired technique such as surface rendering or volume rendering can be selected as the rendering.

S370: Step of displaying image of photoacoustic image data

The computer 150 serving as a second unit (second means) causes the display unit 160 to display the image of the photoacoustic image data obtained in S350. At this time, the computer 150 generates the image of the photoacoustic image data in a surface having the shape as the surface-shape of the object 100 or the similar shape as the surface-shape of the object 100 on the basis of the positional information of the surface of the object 100 obtained in S360, and causes the display unit 160 to display the generated image. The generated image is stored in the storage unit 152 while being associated with information indicating a cross section to be displayed. The information indicating the cross section to be displayed may be in any form of representation such as a coordinate group corresponding to the cross section or a function representing the cross section as long as the position of the cross section can be represented. In a case where an image is generated by projecting a layer having a thickness, the information indicating the cross section may be in any form of representation as long as the position of the layer having the thickness can be represented. It should be noted that the surface having the shape as the surface-shape of the object 100 or the similar shape as the surface-shape of the object typically becomes a surface in parallel to the surface of the object.

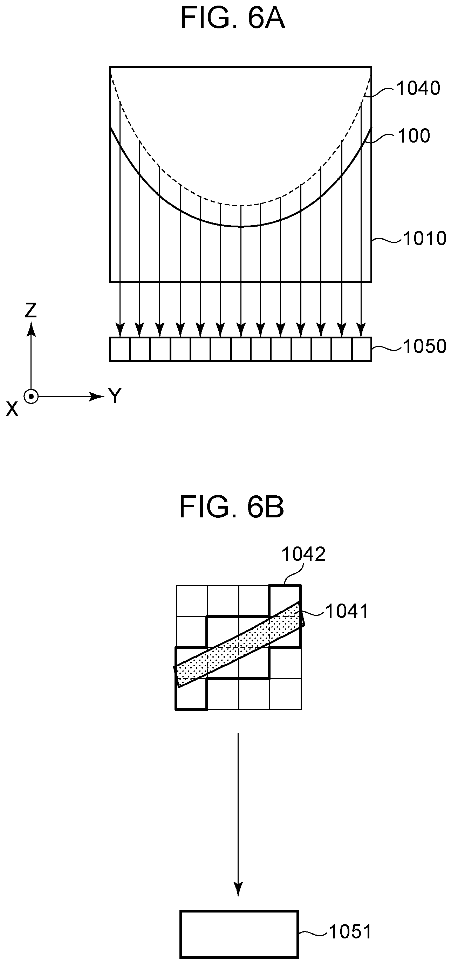

A specific example of the method of displaying the image of the photoacoustic image data will be described with reference to Figs. 6A and 6B, Figs. 7A, 7B, and 7C, and Figs. 8A and 8B.

First, a method of displaying the image of the photoacoustic image data corresponding to a surface 1040 having the shape as the surface-shape of the object 100 or the similar shape as the surface-shape of the object 100 will be described with reference to Fig. 6A. Denoted by reference sign 1050 are virtually defined pixels in a display region of the image of the display unit 160.

The computer 150 determines values of the plurality of pixels 1050 by projecting voxel values of the photoacoustic image data corresponding to the surface 1040 in the lower direction along the plane of the paper (e.g. along the Z-axis direction). Subsequently, the computer 150 transmits the thus determined values of the plurality of pixels 1050 to the display unit 160, and the display unit 160 displays an image constituted by the plurality of pixels 1050.

Here, an example of a generation method for the photoacoustic image data corresponding to the surface 1040 will be described with reference to Fig. 6B. A region 1041 is a region indicating part of the surface 1040. A region 1042 surrounded by a bold line indicates a voxel group used for determining a value of a certain single pixel 1051 among the plurality of pixels 1050. A numeric value obtained by adding all of the voxel values of the photoacoustic image data in the region 1042 to one another is output to the pixel 1051. After the voxels constituting the photoacoustic image data are finely interpolated, the voxels including the region 1041 may be specified, and the value to be output to the pixel 1051 may be determined. It should be noted that a value obtained by dividing the numeric value obtained by adding all of the voxel values of the photoacoustic image data in the region 1042 to one another by the number of voxels may be output to the pixel 1051.

In Figs. 6A and 6B, the example has been described in which the image is generated by projecting the voxel values of the photoacoustic image data corresponding to the surface 1040 in the lower direction along the plane of the paper (Z-axis direction), but the projection may be performed in any direction. The user may specify the projection direction by using the input unit 170. It should be noted that the computer 150 may determine the values corresponding to the display pixels by interpolating the voxel values in a case where the voxels are excessive or deficient with respect to the display pixels.

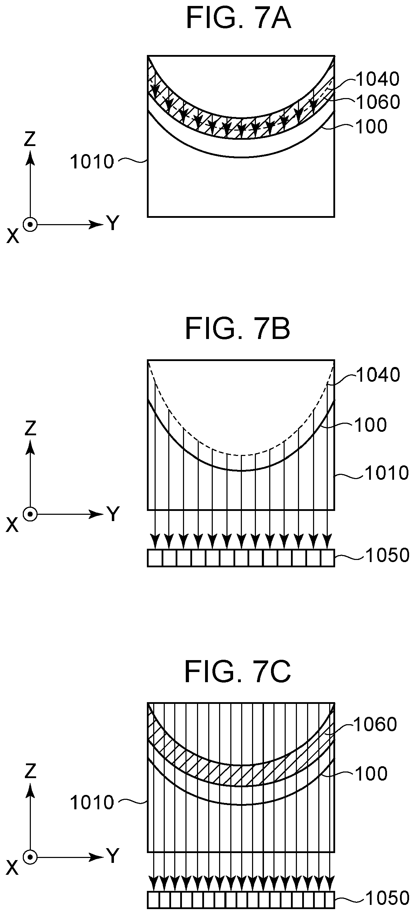

Next, a method of displaying an image obtained by projecting the photoacoustic image data corresponding to a layer 1060 having a thickness, where the thickness has the shape as the surface-shape of the object 100 or the similar shape as the surface-shape of the object 100, will be described with reference to Figs. 7A, 7B, and 7C.

As illustrated in Fig. 7A, the computer 150 projects the photoacoustic image data corresponding to the layer 1060 in the lower direction along the plane of the paper (Z-axis direction) to generate an image on a surface 1040 having the shape as the surface-shape of the object 100 or the similar shape as the surface-shape of the object 100. The thus obtained image on the surface 1040 is projected onto the plurality of pixels 1050 as illustrated in Fig. 7B to generate the image to be displayed on the display unit 160. It should be noted that the display method as illustrated in Fig. 7A and Fig. 7B is equivalent to display of the projecting image in which the photoacoustic image data corresponding to the layer 1060 having the thickness, where the thickness has the shape as the surface-shape of the object 100 or the similar shape as the surface-shape of the object 100, is projected in the lower direction along the plane of the paper (Z-axis direction) as illustrated in Fig. 7C. Any technique such as maximum intensity projection (MIP), minimum intensity projection (MinIP), mean value projection, or median value projection may be adopted as the projection technique. At this time, in a case where the reconstructed voxels are not matched with the layer direction in the thickness, a polar coordinate conversion or the like may be performed to be substantially in parallel to a normal surface such that a region error in the projection direction is reduced. In addition, the reconstructed voxels may be divided, and the projection processing may be executed in an interpolated space. It should be noted that the image of the surface indicating the center of the layer 1060 is generated by the projection in Fig. 7A, but an image of any surface with regard to the layer may be generated by the projection as long as the surface having the surface-shape of the object 100 exists. For example, an image on a boundary close to the surface of the object 100 on the layer 1060 may be generated by the projection, or an image on a boundary far from the surface of the object 100 on the layer 1060 may be generated by the projection.

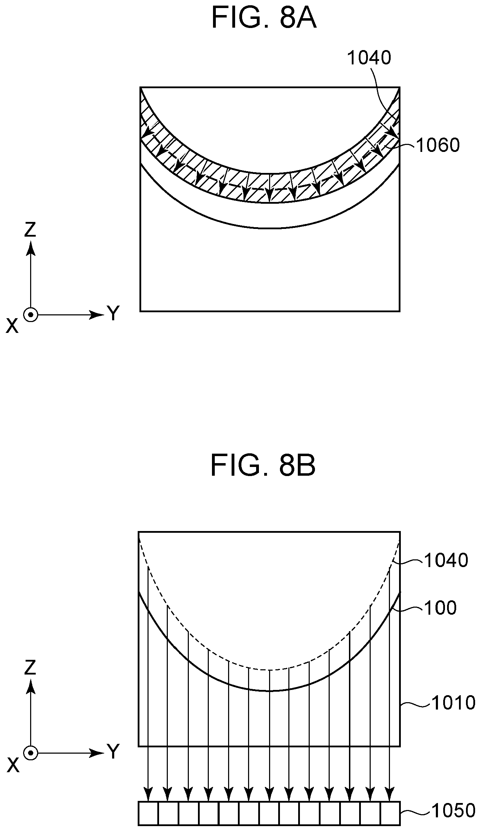

As illustrated in Fig. 8A, the computer 150 may generate an image by projecting the photoacoustic image data corresponding to the layer 1060 having the thickness, where the thickness has the shape as the surface-shape of the object 100 or the similar shape as the surface-shape of the object 100. That is, the computer 150 may project the photoacoustic image data corresponding to the layer 1060 in the normal direction of the surface of the object 100 and generate the image on the surface 1040. Subsequently, as illustrated in Fig. 8B, the computer 150 projects the voxel values on the surface 1040 onto the plurality of pixels 1050 to determine the values of the plurality of pixels 1050. The computer 150 causes the display unit 160 to display the image on the surface 1040 on the basis of the values of the plurality of pixels 1050.

Incidentally, when the image is generated by the projection method illustrated in Fig. 8A and displayed as a two-dimensional image, the two-dimensional image is obtained in which a peripheral part is distorted as compared with a central part of the two-dimensional image. For this reason, the computer 150 may be configured to change the projection direction of the image, that is, positions of the plurality of pixels 1050. The user may also specify the projection direction of the image by using the input unit 170. As a result, it is possible to generate the display image in which the region desired to be observed is not much distorted. That is, the projection direction may be set such that the observed region is located in the central part of the display image.

It should be noted that the example in which the image is displayed by projecting the image on the surface 1040 onto the plurality of virtual pixels 1050 has been described with reference to Figs. 6A and 6B, Figs. 7 to 7C, and Figs. 8A and 8B, but any method may be adopted as long as the image can be displayed. For example, the image on the surface 1040 corresponding to the curved surface may be displayed as a development diagram of the surface 1040 corresponding to the curved surface.

It should be noted that the computer 150 may display an image of the surface having the shape as the surface-shape of the object 100 or the similar shape as the surface-shape of the object 100 in a region of interest (ROI). The user may also manually set the display cross section again by using the input unit 170 while the display image is checked. The computer 150 may also set the ROI on the basis of the instruction of the user using the input unit 170 or evaluation of the voxel values of the photoacoustic image data.

For example, first, the computer 150 may perform rendering display of the photoacoustic image data, and the user may set the ROI with respect to the rendered image by using the input unit 170. The ROI may be specified by the user using the input unit 170 with respect to the rendering image of the image data obtained by another modality. At this time, the user may instruct an arbitrary region with respect to the rendering image and set the region as the ROI. The user may also instruct an arbitrary position with respect to the rendering image and set a predetermined range including the instructed as the ROI. The user may also select a desired region from among a plurality of regions displayed on the display unit 160 and set the region as the ROI. The plurality of regions corresponding to the selection targets may be superimposed on the rendering image.

In addition, the computer 150 may set a region where the voxel values of the photoacoustic image data are within a predetermined numeric value range as the ROI. For example, the computer 150 may set a region where the voxel values of the photoacoustic image data are higher than a predetermined threshold as the ROI. The computer 150 may also set the ROI by similarly evaluating the image data obtained by another modality.

Moreover, a superimposed region of the ROI set by a plurality of methods may be updated as the ROI. It should be noted that a method for the user to specify the ROI by using the input unit 170, a method of setting the ROI on the basis of the image data obtained by another modality, or the like, may be adopted as the setting method for the ROI. For example, the user may set the ROI with respect to the display image of the image data obtained by the other modality by using the input unit 170. The computer 150 can set the ROI in the photoacoustic image corresponding to the ROI in the display image of the other modality.

The computer 150 may perform an analysis with respect to the image on the surface 1040 and cause the display unit 160 to display an analysis result. The analysis mentioned herein refers to statistic processing of a blood vessel density or the number of branches of blood vessels per unit area, a progress direction, an oxygen saturation, or the like.

In addition, the photoacoustic image data and the image data obtained by the other modality may be displayed such that the photoacoustic image data can be compared with the image data in a case where the image data obtained by the other modality exists. It should be noted that the image having the shape as the surface-shape of the object 100 or the similar surface shape as the surface-shape of the object 100 may be displayed with regard to the image data obtained by the other modality in a case where the image having the shape as the surface-shape of the object 100 or the similar shape as the surface-shape of the object 100 is displayed upon display of the image of the photoacoustic image data. In this manner, when the image is displayed in multi modalities, the variation in the visibilities of the images between the modalities is reduced, which contributes to improvement of the diagnosis performance. For example, image data of the respective modalities may be compared and displayed by a display method by displaying the image data in parallel, superimposing, switching, or the like. Those pieces of image data may be superimposed on one another while color maps are changed. It should be noted that, in a case where the shapes of the object 100 in the photoacoustic image data and the image data obtained by the other modality are varied, the computer 150 may alter at least one of the image data to reduce the difference in the shapes. After alteration processing is performed, the computer 150 may generate and display the images of the respective image data on the surface having the shape as the surface-shape of the object 100 or the similar shape as the surface-shape of the object 100 such that the images can be compared with each other. Any related-art technique can be adopted for the alteration processing to reduce the difference in the shapes among the image data.

It should be noted that the computer 150 may define the quasi isosurface of the light fluences on the basis of light fluence distribution information of the irradiation light inside the object 100, and cause the display unit 160 to display the image of the photoacoustic image data on the surface. The computer 150 functioning as a third unit may calculate the light fluence distribution information (three-dimensional spatial distribution of the light fluence) inside the object 100 of the irradiation light.

For example, the computer 150 may calculate the three-dimensional spatial distribution of the light fluence inside the object 100 by solving a light diffusion equation represented by Expression (2).

$$\frac{1}{c}\frac{\partial}{\partial t}\Phi(r,t) = -\mu_a\Phi(r,t) + \nabla\cdot\left(D\nabla\Phi(r,t)\right) + S(r,t) \qquad \cdots \ (2)$$

Where D denotes a diffusion coefficient, $\mu_a$ denotes an absorption coefficient, S denotes an incidence intensity of the irradiation light, $\varphi$ denotes a reaching light fluence, r denotes a position, and t denotes time. Furthermore, the computer 150 can define a surface where substantially equal values of the spatial distribution of the light fluence are continuous as the surface having the shape as the surface-shape of the object 100 or the similar shape as the surface-shape of the object 100 (quasi isosurface of the light fluences). It should be noted that not only a surface constituted by the completely equal value of the light fluence but also a surface where the variation in the light fluences is within a predetermined range may be defined as the quasi isosurface. The user may specify a numeric range where the values of the light fluences can be regarded as substantially equal to one another by using the input unit 170. An order, a coefficient, or the like may be set in accordance with the shape of the object 100 to be imaged with respect to the curved surface or the curved line corresponding to the candidate of the quasi isosurface of the light fluences. As a result, it is possible to reduce processing time used to define the surface having the shape as the surface-shape of the object 100 or the similar shape as the surface-shape of the object 100 (quasi isosurface of the light fluences) on the basis of the spatial distribution of the light fluence.

When the above-described display method is adopted, the image having the small variation in the S/N ratios in the respective positions in the image and the high visibility is displayed. As a result, since the user such as the doctor can conduct the diagnosis by checking the image having the small variation in the image qualities in the image, the diagnostic performance is improved.

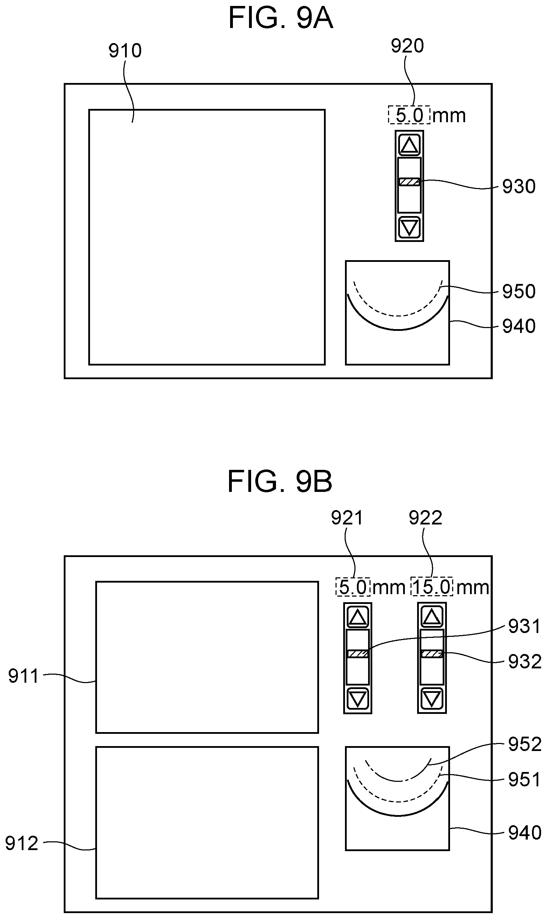

Next, an example of a GUI which realizes a display method according to the present exemplary embodiment will be described. Figs. 9A and 9B are schematic diagrams of the GUI displayed on the display unit 160.

An image on the surface having the shape as the surface-shape of the object 100 or the similar shape as the surface-shape of the object is displayed in an image display area 910.

A depth changing bar 930 is a GUI for setting a position of the cross section of the image to be displayed in the image display area 910. A slider of the depth changing bar 930 corresponds to a distance (depth) from the surface of the object. When the slider is changed, the cross section of the image to be displayed on the image display area 910 is changed. The depth set by the depth changing bar 930 is displayed in a depth display field 920. It should be noted that the user may use the depth display field 920 as the GUI with which depth information can be changed by directly inputting depth information (value of the distance from the surface of the object) or the like in the depth display field 920. In addition, the input unit 170 such as a knob on external hardware or the like may be used as an input interface. For example, when the user operates a wheel of the mouse serving as the input unit 170, the image on the surface having the shape as the surface-shape of the object 100 or the similar shape as the surface-shape of the object may be fed in the normal direction of the surface of the object to be displayed. That is, the images on the plurality of surfaces having the shape as the surface-shape of the object 100 or the similar shape as the surface-shape of the object may be switched and displayed on the basis of the instruction of the user.

A cross section 950 of the image displayed in the image display area 910 is displayed in a reference image display area 940. That is, the cross section 950 corresponding to the depth set by the depth changing bar 930 is displayed in the reference image display area 940. A reference image of the object to be displayed in the reference image display area 940 may be the photoacoustic image data or the rendering image of the image data obtained by the other modality. The reference image of the object to be displayed in the reference image display area 940 may also be an optical image imaged by an optical camera. Moreover, the reference image of the object to be displayed in the reference image display area 940 may be a pattern diagram of the object. When the reference image display area 940 is checked, the user can understand which cross section of the image is currently displayed.

The GUI may be configured such that other display parameters can be set in addition to the depth information. The depth information may be set by any method in addition to the setting method using the depth changing bar 930. For example, when the user specifies a desired position in the reference image display area 940 by clicking the mouse or the like, the computer 150 may define the surface having the shape as the surface-shape of the object 100 or the similar shape as the surface-shape of the object including the position and set the depth information.

Images on a plurality of cross sections having the shape as the surface-shape of the object 100 or the similar shape as the surface-shape of the object may be displayed in parallel as illustrated in Fig. 9B.

A first image at a first depth (first distance from the surface of the object) set by a first depth changing bar 931 is displayed in a first image display area 911. Information indicating the first depth set by the first depth changing bar 931 is displayed in a first depth display field 921.

On the other hand, a second image at a second depth (second distance from the surface of the object) set by a second depth changing bar 932 is displayed in a second image display area 912. Information indicating the second depth set by the second depth changing bar 932 is displayed in a second depth display field 922.

A first cross section 951 of the image displayed in the first image display area 911 is displayed in the reference image display area 940. That is, the first cross section 951 corresponding to the first depth set by the first depth changing bar 931 is displayed in the reference image display area 940.

A second cross section 952 of the image displayed in the second image display area 912 is displayed in the reference image display area 940. The second cross section 952 corresponding to the second depth set by the second depth changing bar 932 is displayed in the reference image display area 940.

It should be noted that the image of the image data obtained by another modality may be mixed with the image of the photoacoustic image data to be displayed. For example, the respective images at the set depths may be superimposed on one another and displayed in the image display area 910 of Fig. 9A. The image of the photoacoustic image data may be displayed in the first image display area 911 of Fig. 9B, and the image of the image data obtained by the other modality may be displayed in the second image display area 912. In this case, the depths corresponding to the respective image display areas may be independently set, or the depths corresponding to the respective image display areas may also be synchronized to be set. A superimposed image obtained from the image of the photoacoustic image data and the image of the image data obtained by the other modality may be displayed in the first image display area 911, and one of the images may be displayed in the second image display area 912. In this manner, an image of image data of any modality may be displayed in any display mode as long as the image on the surface having the shape as the surface-shape of the object 100 or the similar shape as the surface-shape of the object is displayed. It should be noted that the GUI according to the present exemplary embodiment may be able to switch the display from the above-described display to display of an image of image data in which a plane is set as the cross section.

In this manner, the display parameters (depth information and the like) are determined on the basis of the instruction of the user in the GUI according to the present exemplary embodiment, and the image is updated and displayed in accordance with the changed display parameters. As a result, while the image in accordance with the display parameters is checked, the user can set the desired display parameters.

Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The present invention is as set out in the following claims.

**Claims**

1. A display control apparatus configured to cause display means (160) to display an image (910) of photoacoustic image data as volume data derived from a photoacoustic wave generated by light irradiation of an object (100), the display control apparatus comprising:

   first means (150) configured to obtain positional information of a surface of the object (100); and
   second means (150) configured to cause the display means (160) to display the image (910) of the photoacoustic image data on a surface having a shape as the surface-shape of the object (100) or a similar shape as the surface-shape of the object (100) based on the positional information of the surface of the object (100).

2. The display control apparatus according to claim 1, wherein the second means (150) is configured to cause the display means (160) to display a plurality of images (911, 912) of the photoacoustic image data on a plurality of surfaces having the shape as the surface-shape of the object (100) or the similar shape as the surface-shape of the object (100) based on the positional information of the surface of the object (100).

3. The display control apparatus according to claim 2, wherein the second means (150) is configured to switch the plurality of images (911, 912) of the photoacoustic image data on the plurality of surfaces to be displayed on the display means (160) in accordance with an instruction of a user.

4. The display control apparatus according to claim 2, wherein the second means (150) is configured to cause the display means (160) to display the plurality of images (911, 912) of the photoacoustic image data on the plurality of surfaces side by side.

5. The display control apparatus according to any one of claims 1 to 4, wherein the second means (150) is configured to:

   obtain image data obtained by a modality, and
   cause the display means (160) to display a superimposed image obtained by superimposing the image data obtained by the modality on the image of the photoacoustic image data.

6. The display control apparatus according to any one of claims 1 to 4, wherein the second means (150) is configured to:

   obtain image data obtained by a modality, and
   cause the display means (160) to display the image data obtained by a modality and the image of the photoacoustic image data.

7. The display control apparatus according to any one of claims 1 to 6, wherein the second means (160) is configured to:

   determine a layer having a thickness, where the thickness has the shape as the surface-shape of the object (100) or the similar shape as the surface-shape of the object (100), based on the positional information of the surface of the object (100), and
generate the image of the photoacoustic image data by projecting the photoacoustic image data corresponding to the layer in a predetermined direction.

8. The display control apparatus according to claim 7, wherein the second means (150) is configured to:

determine a normal direction of the surface of the object (100) based on the positional information of the surface of the object (100), and
generate the image of the photoacoustic image data by projecting the photoacoustic image data corresponding to the layer in the normal direction of the surface of the object (100).

9. The display control apparatus according to claim 7 or 8, wherein the second means (150) is configured to determine the thickness based on an instruction of a user.

10. The display control apparatus according to any one of claims 1 to 9, wherein the second means (150) is configured to:

cause the display means (160) to display a rendering image of the photoacoustic image data,
set a region of interest based on the rendering image, and
cause the display means (160) to display the image of the photoacoustic image data on the surface having the shape as the surface-shape of the object (100) or the similar shape as the surface-shape of the object in the region of interest.

11. The display control apparatus according to claim 10, wherein the second means (150) is configured to set the region of interest based on an instruction of a user with respect to the rendering image.

12. The display control apparatus according to any one of claims 1 to 11, wherein the first means (150) is configured to obtain the positional information of the surface of the object (100) based on the photoacoustic image data.

13. The display control apparatus according to claim 12, wherein the first means (150) is configured to:

generate smoothed photoacoustic image data by applying a smoothing filter to the photoacoustic image data, and
obtain the positional information of the surface of the object by defining a surface where a variation in image values of the smoothed photoacoustic image data is within a predetermined range as the surface of the object (100).

14. A display control method of displaying an image of photoacoustic image data as volume data derived from a photoacoustic wave generated by light irradiation of object (100), the display control method comprising:

obtaining positional information of a surface of the object (100); and
displaying the image of the photoacoustic image data on a surface having a shape as the surface-shape of the object (100) or a similar shape as the surface-shape of the object (100) based on the positional information of the surface of the object (100).

15. A program that when executed on a computer causes the computer to perform the display control method according to claim 14.

**Patentansprüche**

1. Anzeigesteuervorrichtung, die konfiguriert ist, eine Anzeigeeinrichtung (160) zu veranlassen, ein Bild (910) fotoakustischer Bilddaten als Volumendaten anzuzeigen, die von einer durch Bestrahlung eines Objekts (100) mit Licht erzeugten fotoakustischen Welle abgeleitet sind, wobei die Anzeigesteuervorrichtung umfasst:

eine erste Einrichtung (150), die konfiguriert ist, Positionsinformation einer Fläche des Objekts (100) zu erhalten; und
eine zweite Einrichtung (150), die konfiguriert ist, die Anzeigeeinrichtung (160) zu veranlassen, das Bild (910) der fotoakustischen Bilddaten auf einer Fläche mit einer der Flächenform des Objekts (100) entsprechenden oder ähnlichen Form anzuzeigen, basierend auf der Positionsinformation der Fläche des Objekts (100).

**2.** Anzeigesteuervorrichtung nach Anspruch 1, wobei die zweite Einrichtung (150) konfiguriert ist, die Anzeigeeinrichtung (160) zu veranlassen, mehrere Bilder (911, 912) der fotoakustischen Bilddaten auf mehreren Flächen mit einer der Flächenform des Objekts (100) entsprechenden oder ähnlichen Form anzuzeigen, basierend auf der Positionsinformation der Fläche des Objekts (100).

**3.** Anzeigesteuervorrichtung nach Anspruch 2, wobei die zweite Einrichtung (150) konfiguriert ist, die mehreren auf der Anzeigeeinrichtung (160) anzuzeigenden Bilder (911, 912) der fotoakustischen Bilddaten auf den mehreren Flächen entsprechend einer Benutzeranweisung zu wechseln.

**4.** Anzeigesteuervorrichtung nach Anspruch 2, wobei die zweite Einrichtung (150) konfiguriert ist, die Anzeigeeinrichtung (160) zu veranlassen, die mehreren Bilder (911, 912) der fotoakustischen Bilddaten auf den mehreren Flächen nebeneinander anzuzeigen.

**5.** Anzeigesteuervorrichtung nach einem der Ansprüche 1 bis 4, wobei die zweite Einrichtung (150) konfiguriert ist:

durch eine Modalität erhaltene Bilddaten zu erhalten, und
die Anzeigeeinrichtung (160) zu veranlassen, ein überlagertes Bild anzuzeigen, welches durch Überlagern der durch die Modalität erhaltenen Bilddaten auf dem Bild der fotoakustischen Bilddaten erhalten wird.

**6.** Anzeigesteuervorrichtung nach einem der Ansprüche 1 bis 4, wobei die zweite Einrichtung (150) konfiguriert ist:

durch eine Modalität erhaltene Bilddaten zu erhalten, und
die Anzeigeeinrichtung (160) zu veranlassen, die durch eine Modalität erhaltenen Bilddaten und das Bild der fotoakustischen Bilddaten anzuzeigen.

**7.** Anzeigesteuervorrichtung nach einem der Ansprüche 1 bis 6, wobei die zweite Einrichtung (160) konfiguriert ist:

eine Schicht mit einer Dicke zu bestimmen, wobei die Dicke die der Flächenform des Objekts (100) entsprechende oder ähnliche Form aufweist, basierend auf der Positionsinformation der Fläche des Objekts (100), und
das Bild der fotoakustischen Bilddaten durch Projizieren der der Schicht entsprechenden fotoakustischen Bilddaten in eine vorbestimmte Richtung zu erzeugen.

**8.** Anzeigesteuervorrichtung nach Anspruch 7, wobei die zweite Einrichtung (150) konfiguriert ist:

eine Normalenrichtung der Fläche des Objekts (100) basierend auf der Positionsinformation der Fläche des Objekts (100) zu bestimmen, und
das Bild der fotoakustischen Bilddaten durch Projizieren der der Schicht entsprechenden fotoakustischen Bilddaten in der Normalenrichtung der Fläche des Objekts (100) zu erzeugen.

**9.** Anzeigesteuervorrichtung nach Anspruch 7 oder 8, wobei die zweite Einrichtung (150) konfiguriert ist, die Dicke basierend auf einer Benutzeranweisung zu bestimmen.

**10.** Anzeigesteuervorrichtung nach einem der Ansprüche 1 bis 9, wobei die zweite Einrichtung (150) konfiguriert ist:

die Anzeigeeinrichtung (160) zu veranlassen, ein Render-Bild der fotoakustischen Bilddaten anzuzeigen,
ein Betrachtungsgebiet basierend auf dem Render-Bild einzustellen, und
die Anzeigeeinrichtung (160) zu veranlassen, das Bild der fotoakustischen Bilddaten auf der Fläche mit der der Flächenform des Objekts (100) entsprechenden oder ähnlichen Form im Betrachtungsgebiet anzuzeigen.

**11.** Anzeigesteuervorrichtung nach Anspruch 10, wobei die zweite Einrichtung (150) konfiguriert ist, das Betrachtungsgebiet basierend auf einer Benutzeranweisung hinsichtlich des Render-Bildes einzustellen.

**12.** Anzeigesteuervorrichtung nach einem der Ansprüche 1 bis 11, wobei die erste Einrichtung (150) konfiguriert ist, die Positionsinformation der Fläche des Objekts (100) basierend auf den fotoakustischen Bilddaten zu erhalten.

**13.** Anzeigesteuervorrichtung nach Anspruch 12, wobei die erste Einrichtung (150) konfiguriert ist:

geglättete fotoakustische Bilddaten durch Anwenden eines Glättungsfilters auf die fotoakustischen Bilddaten zu erzeugen, und
die Positionsinformation der Fläche des Objekts durch Definieren einer Fläche, bei der sich eine Veränderung der Bildwerte der geglätteten fotoakustischen Bilddaten innerhalb eines vorbestimmten Bereichs befindet, als die Fläche des Objekts (100) zu erhalten.

14. Anzeigesteuerverfahren des Anzeigens eines Bildes fotoakustischer Bilddaten als Volumendaten, die von einer durch Bestrahlung eines Objekts (100) mit Licht erzeugten fotoakustischen Welle abgeleitet sind, wobei das Anzeigesteuerverfahren umfasst:

Erhalten von Positionsinformation einer Fläche des Objekts (100); und
Anzeigen des Bildes der fotoakustischen Bilddaten auf einer Fläche mit einer der Flächenform des Objekts (100) entsprechenden oder ähnlichen Form basierend auf der Positionsinformation der Fläche des Objekts (100).

15. Programm, welches bei Ausführung auf einem Computer diesen veranlasst, das Anzeigesteuerverfahren nach Anspruch 14 durchzuführen.


**Revendications**

1. Appareil de commande d'affichage configuré pour amener un moyen d'affichage (160) à afficher une image (910) de données d'images photo-acoustiques en tant que données de volume dérivées d'une onde photo-acoustique générée par une exposition à un rayonnement de lumière d'un objet (100), l'appareil de commande d'affichage comprenant :

un premier moyen (150) configuré pour obtenir des informations de position d'une surface de l'objet (100) ; et
un second moyen (150) configuré pour amener le moyen d'affichage (160) à afficher l'image (910) des données d'images photo-acoustiques sur une surface ayant une forme telle que la forme de surface de l'objet (100) ou une forme similaire telle que la forme de surface de l'objet (100) sur la base des informations de position de la surface de l'objet (100).

2. Appareil de commande d'affichage selon la revendication 1, dans lequel le second moyen (150) est configuré pour amener le moyen d'affichage (160) à afficher une pluralité d'images (911, 912) des données d'images photo-acoustiques sur une pluralité de surfaces ayant la forme telle que la forme de surface de l'objet (100) ou la forme similaire telle que la forme de surface de l'objet (100) sur la base des informations de position de la surface de l'objet (100).

3. Appareil de commande d'affichage selon la revendication 2, dans lequel le second moyen (150) est configuré pour commuter la pluralité d'images (911, 912) des données d'images photo-acoustiques sur la pluralité de surfaces pour qu'elles soient affichées sur le moyen d'affichage (160) conformément à une instruction d'un utilisateur.

4. Appareil de commande d'affichage selon la revendication 2, dans lequel le second moyen (150) est configuré pour amener le moyen d'affichage (160) à afficher côte à côte la pluralité d'images (911, 912) des données d'images photo-acoustiques sur la pluralité de surfaces.

5. Appareil de commande d'affichage selon l'une quelconque des revendications 1 à 4, dans lequel le second moyen (150) est configuré pour :

obtenir des données d'images obtenues par une modalité, et
amener le moyen d'affichage (160) à afficher une image superposée obtenue par une superposition des données d'images obtenues par la modalité sur l'image des données d'images photo-acoustiques.

6. Appareil de commande d'affichage selon l'une quelconque des revendications 1 à 4, dans lequel le second moyen (150) est configuré pour :

obtenir des données d'images obtenues par une modalité, et
amener le moyen d'affichage (160) à afficher les données d'images obtenues par une modalité et l'image des données d'images photo-acoustiques.

7. Appareil de commande d'affichage selon l'une quelconque des revendications 1 à 6, dans lequel le second moyen (160) est configuré pour :

   déterminer une couche ayant une certaine épaisseur, la certaine épaisseur ayant la forme telle que la forme de surface de l'objet (100) ou la forme similaire telle que la forme de surface de l'objet (100), sur la base des informations de position de la surface de l'objet (100), et
   générer l'image des données d'images photo-acoustiques par une projection des données d'images photo-acoustiques correspondant à la couche dans une direction prédéterminée.

8. Appareil de commande d'affichage selon la revendication 7, dans lequel le second moyen (150) est configuré pour :

   déterminer une direction normale de la surface de l'objet (100) sur la base des informations de position de la surface de l'objet (100), et
   générer l'image des données d'images photo-acoustiques par une projection des données d'images photo-acoustiques correspondant à la couche dans la direction normale de la surface de l'objet (100).

9. Appareil de commande d'affichage selon la revendication 7 ou 8, dans lequel le second moyen (150) est configuré pour déterminer l'épaisseur sur la base d'une instruction d'un utilisateur.

10. Appareil de commande d'affichage selon l'une quelconque des revendications 1 à 9, dans lequel le second moyen (150) est configuré pour :

    amener le moyen d'affichage (160) à afficher une image de rendu des données d'images photo-acoustiques, définir une région d'intérêt sur la base de l'image de rendu, et
    amener le moyen d'affichage (160) à afficher l'image des données d'images photo-acoustiques sur la surface ayant la forme telle que la forme de surface de l'objet (100) ou la forme similaire telle que la forme de surface de l'objet dans la région d'intérêt.

11. Appareil de commande d'affichage selon la revendication 10, dans lequel le second moyen (150) est configuré pour définir la région d'intérêt sur la base d'une instruction d'un utilisateur par rapport à l'image de rendu.

12. Appareil de commande d'affichage selon l'une quelconque des revendications 1 à 11, dans lequel le premier moyen (150) est configuré pour obtenir les informations de position de la surface de l'objet (100) sur la base des données d'images photo-acoustiques.

13. Appareil de commande d'affichage selon la revendication 12, dans lequel le premier moyen (150) est configuré pour :

    générer des données d'images photo-acoustiques lissées par une application d'un filtre de lissage aux données d'images photo-acoustiques, et
    obtenir les informations de position de la surface de l'objet par une définition d'une surface au niveau de laquelle une variation de valeurs d'images des données d'images photo-acoustiques lissées s'inscrit dans une plage prédéterminée en tant que la surface de l'objet (100).

14. Procédé de commande d'affichage consistant à afficher une image de données d'images photo-acoustiques telles que des données de volume dérivées d'une onde photo-acoustique générée par une exposition à un rayonnement de lumière d'un objet (100), le procédé de commande d'affichage comprenant les étapes consistant à :

    obtenir des informations de position d'une surface de l'objet (100) ; et
    afficher l'image des données d'images photo-acoustiques sur une surface ayant une forme telle que la forme de surface de l'objet (100) ou une forme similaire telle que la forme de surface de l'objet (100) sur la base des informations de position de la surface de l'objet (100).

15. Programme qui, lorsqu'il est exécuté sur un ordinateur, amène l'ordinateur à mettre en œuvre le procédé de commande d'affichage selon la revendication 14.

## FIG. 1A

## FIG. 1B

# FIG. 2

# FIG. 3A

# FIG. 3B

# FIG. 4

150

# FIG. 5

START

SET CONTROL PARAMETER ~ S310

MOVE PROBE TO SPECIFIED POSITION ~ S320

LIGHT IRRADIATION ~ S330

RECEIVE PHOTOACOUSTIC WAVE ~ S340

GENERATE PHOTOACOUSTIC IMAGE DATA ~ S350

OBTAIN POSITIONAL INFORMATION OF SURFACE OF OBJECT ~ S360

DISPLAY IMAGE OF PHOTOACOUSTIC IMAGE DATA ~ S370

END

# FIG. 6A

# FIG. 6B

## FIG. 7A

## FIG. 7B

## FIG. 7C

## FIG. 8A

1040

1060

Z

X Y

## FIG. 8B

1040

100

1010

1050

Z

X Y

# FIG. 9A

910    920

5.0 mm

930

950
940

# FIG. 9B

921    922

5.0 mm    15.0 mm

931
932

911

952
951

912

940

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013233386 A

- US 2014196544 A1